# EUROPÄISCHE PATENTANMELDUNG

(11) **EP 4 714 389 A1**
(43) Veröffentlichungstag der Anmeldung: **25.03.2026**
(21) Anmeldenummer: 25202412.0
(22) Anmeldetag: 16.09.2025
(51) Int. Cl.: A61B 50/20, A61B 90/90, A61B 90/00

(54) **ÖFFNUNGSSYSTEM, GREIFSYSTEM UND VERFAHREN**

(30) Priorität: 19.09.2024 DE 102024126886
(71) Anmelder: Aesculap AG, 78532 Tuttlingen (DE)
(72) Erfinder: JANSEN-TROY, Arne, 78333 Stockach (DE); EBERHARDT-FONSECA, André-Manuel, 70186 Stuttgart (DE); MAAS, Allan, 78467 Konstanz (DE); KOLLER, Tobias, 78532 Tuttlingen (DE)
(74) Vertreter: Tergau & Walkenhorst Intellectual Property GmbH

(57) **Zusammenfassung**

Öffnungssystem (1) zum Öffnen und/oder Entriegeln eines medizinischen, insbesondere chirurgischen, Instrumentes (38) mit Ringgriffen, umfassend
- eine Basis (2);
- zwei auf der Basis (2) angeordnete und mittels eines Aktors (12) gegeneinander verschiebbare Achsen (3, 5), die jeweils auf flächigen Grundelementen (9a, 9b) angeordnet sind, welche zur Basis einen unterschiedlichen Abstand aufweisen;
- zwei Schwenk-Klemmvorrichtungen (13a, 13b), welche jeweils ein Klemmelement (8a, 8b) zum Klemmen eines auf eine Achse (3,5) eingefädelten Ringgriffes mit dem Grundelement (9a, 9b) umfassen.

## Beschreibung

Die Erfindung betrifft ein Öffnungssystem und ein Greifsystem mit einem derartigen Öffnungssystem. Sie betrifft auch ein Verfahren zum Betreiben des Greifsystems.

Das Tragen der Sterilgut-Container und das Anheben bzw. Ein- und Ausladen in die Sterilgut-Container-Transportwagen und Regale auf sehr hohe oder tiefe Positionen sowie das Entnahmen der Siebkörbe aus den Containern stellen nach wie vor ein ermüdendes und auf lange Zeit gesundheitsschädliches (insbesondere für den Rücken) Arbeiten für die Angestellten einer Aufbereitungseinheit für Medizinprodukte (AEMP) dar. Auch das Öffnen bzw. Entriegeln von medizinischen Instrumenten von Hand ist eine anstrengende und ermüdende Tätigkeit. Zum automatisierten Transport sowie der Be- und Entladung und dem Handling der Sterilgut-Container und Siebkörbe wurden daher bereits mehrere Lösungen technische Lösungen vorgeschlagen.

Forschungsansätze zu einer Instrumentenseparierung basierend auf einer Objekterkennung medizinischer Instrumente sind in der Praxis stark eingeschränkt. Das Vorhandensein von CAD-Daten aller zu sortierender Instrumente oder die Annahme, dass die Instrumente bei Ankunft an einer AEMP sauber und in einem geschlossenen Zustand vorliegen, sind unrealistisch für eine Instrumentenseparierung auf der unreinen Seite. Ein Greifer-System für den Sortierprozess der medizinischen Instrumente aus einem Siebkorb heraus muss dazu in der Lage sein, übereinander gestapelte Instrumente separat voneinander greifen zu können. Zudem sollten die medizinischen Instrumente erfolgreich an der gewünschten Stelle abgelegt werden können, um unnötige Prozessschritte zu vermeiden beziehungsweise den nächsten Prozessschritt nicht zu behindern.

Aus der EP 3 923 850 A1 ist eine Entsperrvorrichtung zum Entsperren von zusammenwirkende Sperrelemente umfassenden Sperreinrichtungen von mindestens zwei medizinischen Ringinstrumenten bekannt, wobei die Entsperrvorrichtung einen ersten Aufnahmekörper mit mindestens zwei Ringaufnahmen zum Aufnehmen von ersten Ringe der mindestens zwei Ringinstrumente umfasst, und wobei die Entsperrvorrichtung einen Entsperrkörper mit mindestens zwei mit zweiten Ringen der mindestens zwei Ringinstrumente zusammenwirkenden Entsperrelementen umfasst, wobei der Entsperrkörper und der Aufnahmekörper relativ zueinander in einer Betätigungsrichtung bewegbar angeordnet sind. Die medizinischen Instrumente müssen zum Einlegen in die Entsperrvorrichtung so geöffnet werden, dass diese von der Entsperrvorrichtung aufgenommen werden können, da sie nicht an den Öffnungswinkel der medizinischen Instrumente anpassbar ist.

Der Erfindung liegt daher die Aufgabe zugrunde, ein Öffnungssystem zum automatisierten Öffnungen und/oder Entriegeln eines medizinischen Instrumentes bereitzustellen. Der Erfindung liegt weiterhin die Aufgabe zugrunde ein Greifsystem bereitzustellen, welches die automatisierte Handhabung von medizinischen Instrumenten durchführt. Der Erfindung liegt weiterhin die Aufgabe zugrunde, ein entsprechendes Betriebsverfahren bereitzustellen.

In Bezug auf das Öffnungssystem wird die oben genannte Aufgabe erfindungsgemäß gelöst durch ein Öffnungssystem mit den Merkmalen von Anspruch 1. Das Öffnungssystem umfasst eine Basis und zwei auf der Basis angeordnete und mittels eines Aktors gegeneinander verschiebbare Achsen, die jeweils auf flächigen Grundelementen angeordnet sind, welche zur Basis einen unterschiedlichen Abstand aufweisen. Das Öffnungssystem umfasst weiterhin zwei Schwenk-Klemmvorrichtungen, welche jeweils ein Klemmelement zum Klemmen eines auf eine Achse eingefädelten Ringgriffes mit dem Grundelement umfassen.

Vorteilhafte Ausgestaltungen der Erfindung sind Gegenstand der Unteransprüche.

Die Erfindung geht von der Überlegung aus, dass insbesondere medizinische Instrumente mit Ringgriffen, welche gewöhnlich mit Hilfe der Griffe ein- und aufgeklappt werden, einen bestimmten Öffnungswinkel zur weiteren Verarbeitung, insbesondere zur Reinigung und Sterilisation, eingestellt haben müssen. Zudem weisen diese medizinischen Instrumente gewöhnlich einen Verriegelungsmechanismus auf, welcher gelöst werden muss, um das medizinische Instrument mit einem bestimmten Öffnungswinkel einzustellen.

Wie nunmehr erkannt wurde, können diese Aufgaben mit Hilfe eines automatisierten Öffnungssystem durchgeführt werden, welches sowohl das Öffnen als auch das Entriegeln des medizinischen Instrumentes ohne manuelles Eingreifen selbständig durchführt. Ein solches Öffnungssystem kann in ein Greifsystem integriert werden, was eine automatisierte Verarbeitung der medizinischen Instrumente von der Anlieferung der Sterilgut-Container bis zur Ausgabe der medizinischen Instrumente auf der reinen Seite durch die Reinigungs- und Desinfektionsgeräte (RDGs) ermöglicht.

Das jeweilige medizinische Instrument ist insbesondere ein chirurgisches Instrument, kann aber auch ein orthopädisches oder neurologisches Instrument sein.

Vorteilhafterweise ist die jeweilige Schwenk-Klemmvorrichtung als Schwenk-Klemmzylinder ausgebildet. Der Schwenk-Klemmzylinder kann nach pneumatischen oder hydraulischem Prinzip die Schwenkung und Bewegung des Klemmelementes in Richtung der Basis und in entgegengesetzter Richtung ermöglichen. Alternativ kann dazu auch ein Elektroantrieb vorgesehen sein.

Die jeweilige Schwenk-Klemmvorrichtung umfasst vorteilhafterweise das Grundelement, welches mit Hilfe eines Antriebs verfahrbar ist, um eine Öffnungsrichtung des Ringinstrumentes in eine jeweils untere oder obere Richtung zu ermöglichen.

Die Schwenk-Klemmzylinder können kraftgesteuert ausgebildet sein, bevorzugt ist aber zusätzlich ein Kraftmesssensor vorgesehen, welcher zur Steuerung verwendet wird. Die Steuerung kann über den Druck im System oder durch einen Kraftmomentensensor, besonders bevorzugt aber über ein, in die jeweilige Grundfläche eingebrachten Dehnmessstreifen erfolgen. Die Information zur Verfahrrichtung der Schwenk-Klemmvorrichtung bzw. des Schwenk-Klemmzylinders erhält die Steuer- und Regeleinheit aus den Messungen des vorgeschlagenen Kraftsensors und/oder durch das optische System.

Der Niveauunterschied der beiden Grundflächen zueinander, d.h. auch die Differenz ihres Abstandes zur Basis, beträgt bevorzugt mindestens 5 mm, besonders bevorzugt 10 mm bis 15 mm.

In einer bevorzugten Ausführungsform ist auf der Basis eine Schiene angeordnet, auf welcher ein Schlitten verfahrbar ist, auf welchem eine der Achsen und eine Schwenk-Klemmvorrichtung angeordnet sind. Durch Verfahren des Schlittens kann der Abstand der beiden Achsen zueinander variiert werden, sodass Öffnungs- und Schließbewegungen des medizinischen Instruments möglich sind, wenn die Ringgriffe jeweils in Eingriff mit einer Achse und festgeklemmt sind.

Der Schlitten ist bevorzugt mittels einer durch den Aktor drehbaren Spindel entlang der Schiene verfahrbar.

Der Aktor des Öffnungssystems umfasst bevorzugt einen Elektroantrieb und ein Getriebe.

In Bezug auf das Greifsystem wird die oben genannte Aufgabe erfindungsgemäß gelöst durch ein Greifsystem mit den Merkmalen von Anspruch 7. Das Greifsystem umfasst einen Greifer zum Anheben und Ablegen des medizinischen Instruments, eine Objekterkennungsvorrichtung und ein oben beschriebenes Öffnungssystem sowie eine Steuer- und Regeleinheit. Die Objekterkennungsvorrichtung ist zum Erkennen eines medizinischen Instrumentes mit Ringgriffen ausgebildet, wobei das Greifsystem ausgebildet ist, das medizinische Instrument mit Ringgriffen in das Öffnungssystem einzulegen.

Die Objekterkennungsvorrichtung weist dazu vorteilhafterweise einen Klassifikator auf, welcher insbesondere als neuronales Netz ausgebildet ist, zum Klassifizieren des medizinischen Instrumentes in die Klassen "weist Ringriffe auf" und "weist keine Ringgriffe auf". Nur, wenn das medizinische Instrument Ringgriffe aufweist, wird es in das Öffnungssystem eingelegt.

Der Klassifikator ist bevorzugt ausgebildet, einen Verriegelungsmechanismus des medizinischen Instruments zu erkennen, wobei die Steuer- und Regeleinheit den Greifer aufgrund des erkannten Verriegelungsmechanismus derart ansteuert, dass dieses in einer Entriegelungsorientierung in das Öffnungssystem eingelegt wird.

Das Greifsystem umfasst vorteilhafterweise eine 6-DOF-Einheit, an welcher das Öffnungssystem, insbesondere die Basis des Öffnungssystems, montiert ist.

Das Greifsystem umfasst vorteilhafterweise einen 6-DOF-Knickarmroboter, an welchem der Greifer montiert ist. Mit Hilfe der 6-DOF-Einheit und des 6-DOF-Knickarms ist eine sehr flexible Positionierung und Ausrichtung des Greifers zum Öffnungssystem möglich.

In Bezug auf das Verfahren wird die oben genannte Aufgabe erfindungsgemäß gelöst durch ein Verfahren mit den Merkmalen von Anspruch 12. Das Verfahren umfasst die Schritte des Greifens eines medizinischen Instrumentes und Ablegen des Instrumentes auf einer Ablagefläche und/oder Positionieren des Instrumentes vor einer Referenzfläche, des optischen Erfassens der Konturen des medizinischen Instrumentes und Erkennen, ob das medizinische Instrument Ringgriffe aufweist, und sofern dies der Fall ist, Platzieren des medizinischen Instrumentes in dem Öffnungssystem, und Öffnungen und/oder Entriegeln des medizinischen Instrumentes durch das Öffnungssystem sowie erneutes Greifen des medizinischen Instrumentes und Ablegen des medizinischen Instrumentes an einer vorgegeben Stelle.

Der Schritt des Greifens eines medizinischen Instrumentes und Ablegen des medizinischen Instrumentes auf einer Ablagefläche und/oder Positionieren des medizinischen Instrumentes vor einer Referenzfläche umfasst den Schritt, dass das medizinische Instrument zur korrekten Erkennung der Umrisse/Geometrie ohne Ablage vor einer Referenzfläche (z.B. einfarbig grüne Fläche) positioniert wird, während es noch vom Roboterarm bzw. Greifer gehalten wird/bleibt.

Das medizinische Instrument kann dann entweder danach abgelegt und von einem anderen Roboterarm bzw. Greifer (oder demselben) wieder aufgenommen werden (ggf. mit korrigierter Greifposition an der berechneten Greifkoordinate), oder es kann direkt ohne Ablage an einen anderen Roboterarm übergeben werden, der das medizinische Instrument an der Greifkoordinate greift/aufnimmt.

Vorteilhafterweise werden die Konturen des medizinischen Instrumentes mit Hilfe eines Kantenerkennungsalgorithmus erkannt. Besonders bevorzugt wird hierzu der Canny-Edge-Algorithmus verwendet.

In einer bevorzugten Ausführung werden mit Hilfe der Objekterkennungsvorrichtung Merkmale auf dem medizinischen Instrument erkannt und darauf basierend wird ein Output generiert. Diese Merkmale sind insbesondere Datamatrixcodes, Barcodes, Beschriftung etc. Auf diese Weise können bereits auf der unreinen Seite die Artikelnummer und Lotnummer erkannt werden und schon auf der unreinen Seite kann über den Abgleich der Packliste gemeldet werden, ob alle medizinischen Instrumente vorhanden sind. Die Meldung wird bevorzugt im Produktmanagementsystem widergespiegelt, d.h. über ein Interface zum User, oder es wird automatisch nachgelegt. Dies ist derzeit erst beim Packen möglich.

Das Greifen erfolgt entlang eines vorgegebenen Rasters. Dazu sind vorteilhaft Punkte in einer Fläche in x- und y- und danach weiter in z-Richtung vorgegeben, wenn eine Fläche vollständig abgefahren wurde. Am Ende erfolgt eine Kontrolle, ob das Sieb geleert wurde. Das bedeutet, dass zunächst eine erste Ebene abgefahren wird (d.h. die oberste), und anschließend eine zweite, tieferliegende Ebene, damit beim Verfahren in x- und y-Richtung der Greifer kein medizinisches Instrument verschiebt und dadurch mit anderen verkeilt und/oder das medizinische Instrument beschädigt.

Die Vorteile der Erfindung liegen insbesondere darin, dass das Öffnungssystem eine automatisierte Öffnung und Entriegelung von medizinischen Instrumenten mit Ringgriffen ermöglicht, wodurch das medizinische Personal entlastet wird.

Mit dem Greifsystem wird durch das Schließen einer bislang bestehenden Automatisierungslücke eine vollständig automatisierte Handhabung von Instrumenten von der unreinen bis zur reinen Seite ermöglicht. Das Greifsystem kann auf der unreinen Seite einer AEMP (Aufbereitungseinheit für Medizinprodukte) und als Teilsystem einer robotischen Instrumentenvereinzelung Verwendung finden, um alle Prozesse innerhalb des Sterilgutkreislaufs von der Anlieferung der kontaminierten medizinischen Instrumente in einem Sterilgut-Container bis zu deren Reinigung und Desinfektion zu automatisieren.

Durch das beschriebene Verfahren können medizinische Instrumente der Klasse "kein Ringgriff und "Ringgriff' gezielt von einer Ablagefläche aufgenommen werden, um anschließend klassenspezifisch abgelegt und weiter manipuliert zu werden. Medizinische Instrumente mit einem Ringgriff können automatisiert im idealen Öffnungswinkel entsprechend den Vorgaben für das jeweilige medizinische Instrument geöffnet werden. Diese Vorgaben sind digital hinterlegt und werden dann beim Erkennen der Instrumentengeometrie an die Steuerung der Achsen weitergegeben. Die medizinischen Instrumente können so für eine optimale Reinigung insbesondere automatisierte Reinigung vorbereitet werden.

Ein Ausführungsbeispiel der Erfindung wird anhand einer Zeichnung näher erläutert. Darin zeigen in stark schematisierter Darstellung:
- FIG. 1: ein Greifsystem in einer bevorzugten Ausführungsform;
- FIG. 2: ein Öffnungssystem in einer bevorzugten Ausführungsform;
- FIG. 3: eine Ablagefläche mit einer Schere;
- FIG. 4: die Schere mit berechneten geometrischen Größen, und
- FIG. 5: ein Ablaufdiagramm eines Verfahrens in einer bevorzugten Ausführungsform.

Gleiche Teile sind in allen Figuren mit denselben Bezugszeichen versehen.

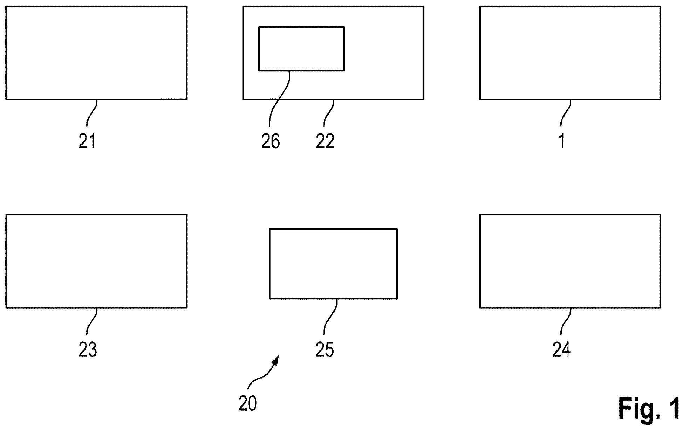

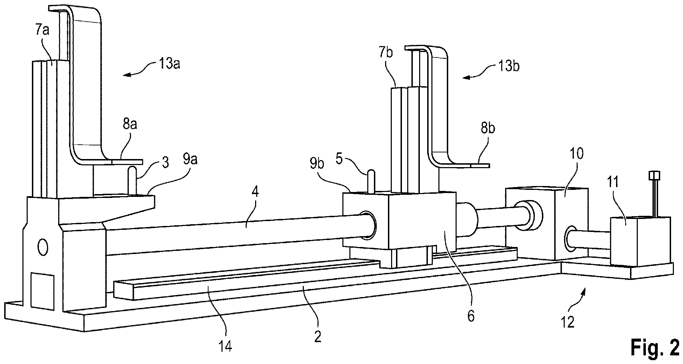

Ein in FIG. 1 schematisch dargestelltes Greifsystem 20 umfasst einen elektromagnetischen Greifer 21 zum Anheben und Ablegen eines medizinischen, insbesondere chirurgischen, Instruments 38. Das Greifsystem 20 weist weiterhin eine Objekterkennungsvorrichtung 22 auf sowie ein Öffnungssystem 1, welches unten im Zusammenhang mit FIG. 2 beschrieben wird. Das Greifsystem 20 umfasst weiterhin eine 6-DOF-Einheit, an welcher das Öffnungssystem 1 montiert ist, sowie einen 6-DOF-Knickarmroboter 24, an welchem der Greifer 21 montiert ist.

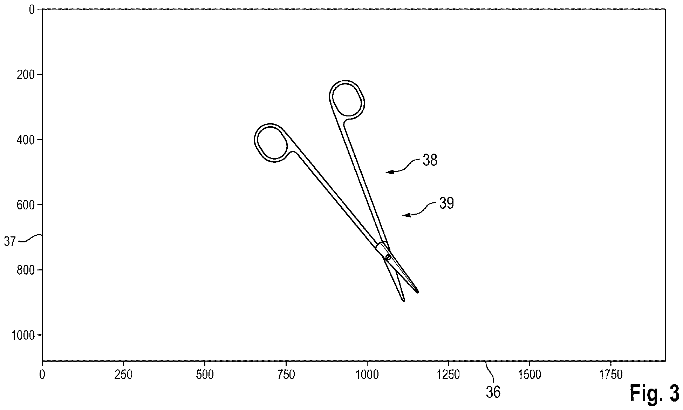

Die Objekterkennungsvorrichtung 22 ist zum Erkennen eines medizinischen Instrumentes 38 (siehe FIG. 3), welches mit Ringgriffen versehen ist, ausgebildet und weist dazu einen Klassifikator 26 auf, welcher vorliegend als neuronal Netz ausgebildet ist, welches mit Abbildungen von medizinischen Instrumenten mit Ringriffen und ohne Ringgriffe durch überwachtes Lernen trainiert wurde. Das Greifsystem 20 ist dazu ausgebildet, das medizinische Instrument 38 mit Ringgriffen in das Öffnungssystem 1 zu legen. Das Greifsystem 20 weist eine Steuer- und Regeleinheit 25 auf, welche als zentrale Steuer- und Regeleinheit ausgebildet sein kann oder mehrere, den einzelnen Systemkomponenten zugeordnete Steuer- und Regeleinheiten umfassen kann.

In FIG. 2 ist ein Öffnungssystem 1 für Ringinstrumente in einer bevorzugten Ausführungsform dargestellt. Das Öffnungssystem weist eine Basis 2 auf. Die Basis 2 kann an einer, vorzugsweise räumlich und automatisiert verfahrbaren 6 DOF-Einheit (6 Degree-of-freedom Einheit) montiert sein (nicht dargestellt). Alternativ kann das Öffnungssystem 1 stationär aufgebaut sein, wobei sich das Öffnungssystem 1 dann vorzugsweise innerhalb der Zelle und im Arbeitsbereich des elektromagnetischen Greifers 21 befindet.

Das Öffnungssystem 1 umfasst zwei automatisiert relativ zueinander verschiebbare Achsen 3, 5, welche vorliegend zylinderförmig ausgebildet sind und sich auf der Basis 2 befinden. Die beiden Achsen 3, 5 lassen sich auf die Distanz beider Ringgriffe bzw. der beiden Schenkel eines zu manipulierenden Ringinstrumentes 38 (siehe FIG. 3) in dessen aktuellen Zustand (ggf. dem geschlossenen und damit dem ggf. arretierten Zustand oder dem vollständig geöffneten Zustand oder Positionen zwischen diesen Endstellungen) einstellen (die Berechnung des Abstandes beider Ringgriffe wird unten erläutert 2), welches durch den oben beschriebenen elektromagnetischen Greifer 21 bewegt bzw. positioniert werden kann.

Eine der beiden Achsen 3, 5 kann hierbei als nicht verschiebbar relativ zur Basis 2 ausgebildet sein und damit eine relative ortsfeste Position beibehalten. Da insbesondere medizinische Instrumente wie Arterienklemmen einen Arretierungsmechanismus besitzen, ist das Öffnungssystem 1 ausgebildet, das Öffnen der arretierten Ringgriffe zu ermöglichen.

Dazu ist die mit einem Aktor 12, welcher vorliegend als Spindelantrieb 4 ausgebildet ist, positionierbare Achse 5 ist auf einem Schlitten 6 linear auf einer auf der Basis 2 befestigte Schiene 14 verschiebbar. Vorliegend ist ein Elektroantrieb 11 vorgesehen, welcher mit einem Encoder eine Spindel 4 über ein Getriebe 10 antreibt. Mittels des Encoders kann über den Elektroantrieb 11 die gewünschte Position der beiden Achsen 3, 5 zueinander über die Drehung der Spindel 4 eingestellt werden.

Das Öffnungssystem 1 umfasst zwei Schwenk-Klemmvorrichtungen 13a, 13 b, welche vorliegend als Schwenk-Klemmzylinder 7a, 7b ausgebildet sind und die geschlossen werden, sobald das medizinische Instrument mit den beiden Ringgriffen auf den beiden Achsen 3, 5 aufgefädelt ist. Über zwei an den Schwenk-Klemmzylindern 7a, 7b montierte Klemmelemente 8a, 8b werden die beiden Ringgriffe auf den Achsen 3, 5 an die Grundflächen der Achsenaufnahme 9a, 9b gepresst. Ein Höhenversatz der Grundflächen 9a, 9b zueinander sorgt zusammen mit der Klemmung beider Ringgriffringe auf den unterschiedlichen Niveaus der Grundflächen 9a, 9b für ein Öffnen der Arretierung bei beispielsweise Arterienklemmen.

Die beiden Griffringe werden in zwei zueinander parallelen Ebenen zwischen je einer Grundfläche 9a oder 9b und einem Klemmelement 8a oder 8b geklemmt. Da die beiden Klemmebenen zueinander parallel, aber auf unterschiedlicher Höhe über der Basis angeordnet sind, werden die beiden Branchen/Schenkel der Griffelemente gegeneinander elastisch verformt, sodass eine zwischen diesen Brachen vorgesehene Sperre (bzw. Arretierung) außer Eingriff gebracht wird. Sobald die medizinischen Instrumente 38 geklemmt sind und damit die Arretierung geöffnet ist, können die beiden Achsen 3, 5 relativ zueinander auseinandergefahren und damit der optimale Öffnungswinkel (beispielsweise 90°) eingestellt werden. Dies ermöglicht im Anschluss eine optimale Reinigung. Es wird vorgeschlagen, dass zusätzlich zur Erkennung bzw. der Navigation zum Auffädeln der Ringinstrumente über eine Speicherprogrammierbare Steuerung (nicht dargestellt) der optimale Öffnungswinkel zu jedem Ringinstrument bzw. Artikel hinterlegt und eingestellt wird.

Die Klemmelemente 8a, 8b werden vorteilhafterweise über die Schwenk-Klemmzylinder 7a, 7b so geschwenkt, dass sich die Ringe der medizinischen Instrumente 38 auf die Achsen 3, 5 aufschieben lassen. Anschließend wird der jeweilige Schwenk-Klemmzylinder zurückgeschwenkt (Position wie in Figur 1 dargestellt) und klemmt anschließend den Ring des medizinischen Instruments 38 gegenüber der jeweiligen Grundfläche 9a oder 9b. Die Schwenk-Klemmzylinder 7a, 7b sind in der Regel kraftgesteuert, es wird aber vorgeschlagen, zusätzlich einen Kraftmesssensor zur Steuerung zu verwenden - dies kann über den Druck im System oder durch einen Kraftmomentensensor, idealerweise aber über ein, in die jeweilige Grundfläche 9a, 9b eingebrachten Dehnmessstreifen erfolgen. Es wird vorgeschlagen eine Niveauunterschied der beiden Grundflächen 9a und 9b zueinander vorzusehen, der mindestens 5 mm, besser jedoch 10 mm bis 15 mm beträgt.

Für die Berechnung des Abstandes der beiden Ringgriffe nach der Positionierung auf der Ablagefläche und die anschließende Manipulation der Instrumentenausrichtung ist optimalerweise ein dunkler Hintergrund für die Objekterkennung und insbesondere bei der Kantenerkennung mit dem aus dem Stand der Technik entsprechenden Canny-Algorithmus vorteilhaft. Ein heller Hintergrund führt neben einem mangelnden Kontrast bei stark reflektierenden Instrumenten zu einer Überbelichtung der Kamera bei einigen Aufnahmen.

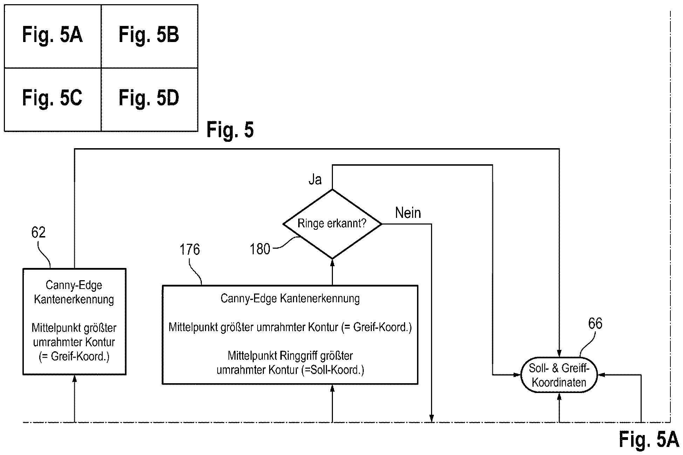

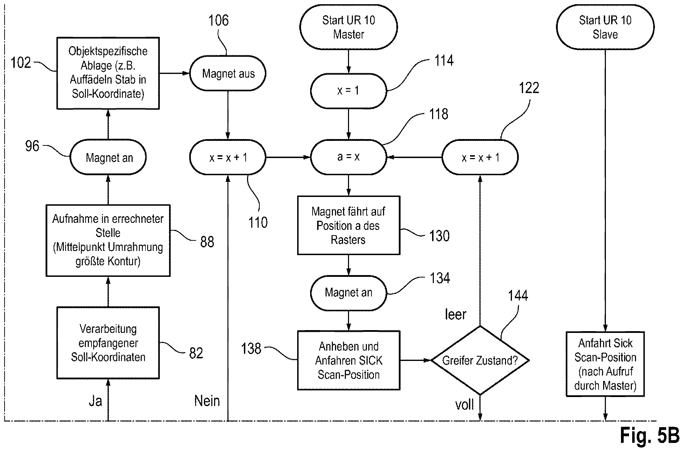

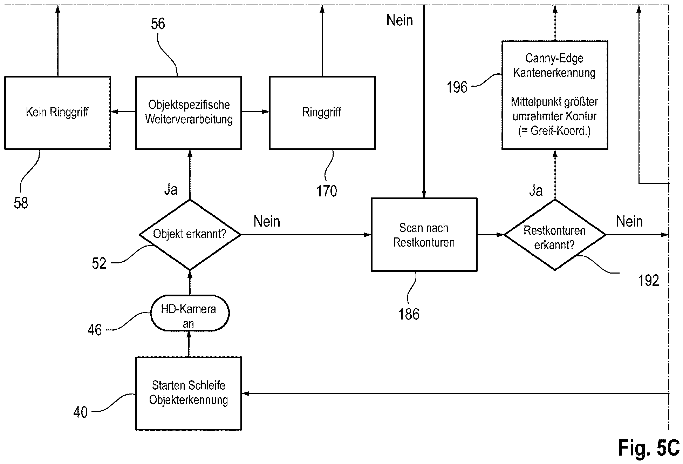

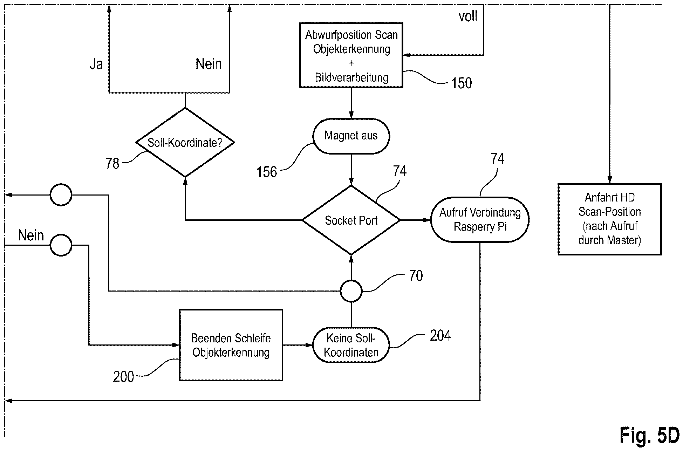

In FIG. 5 ist schematisch ein Ablaufdiagramm eines Verfahrens zum Betrieben des Greifsystems 20 in einer bevorzugten Ausführung dargestellt. Grundsätzlich ist das Greifen verschiedener medizinischer Instrumente 38 notwendig, wobei diese in die Instrumentenklassen "Ringgriff und "kein Ringgriff als Prämisse für das zugrunde liegende Verfahren eingeteilt werden. Medizinische Instrumente 38 verfügen nämlich grundsätzlich über einen Ringgriff (bspw. Schere) oder über einen nicht mit Ringgriffen verbundenen Mechanismus (bspw. Pinzette), durch den sie geöffnet und geschlossen werden können. Die Kinematik zum Öffnen der medizinischen Instrumente 38 ist maßgeblich für ein erfolgreiches automatisiertes Separieren und Reinigen medizinischer Instrumente 38.

Auf einer Ablagefläche wird durch einen zuvor erfolgten "Griff in die Kiste" über ein starres Raster (abhängig von der Größe des Siebkorbs) mit einem elektromagnetischen Greifer 21 ein medizinisches Instrument 38 abgelegt. Dieser Schritt bis zur Ablage auf der Ablagefläche erfolgt ohne eine gezielte Koordinatenberechnung der Greifkoordinate. Um ein medizinisches Instrument 38 jedoch an einer hierfür definierten Position in einer spezifischen Ausrichtung abzulegen, ist eine Koordinatenberechnung der Greifkoordinate sowie der Kenntnis der Ringe bei einem medizinischen Instrument 38 der Klasse "Ringgriff' notwendig, um dieses über den oben beschriebenen Mechanismus zu öffnen.

Die Koordinatenerfassung kann auch ohne Ablegen des medizinischen Instruments 38 erfolgen, indem das medizinische Instrument 38 im noch gegriffenen Zustand vor einer Referenzfläche positioniert wird.

Das Verfahren beginnt zur Objekterkennung in Schritt 40 die Schleife der Objekterkennung gestartet wird. In einem ersten Terminal 46 wird ein Scan der Ablagefläche durch eine Aufnahme der Ablagefläche mittels einer Webcam aus der Vogelperspektive durchgeführt. Die Bildebene ist hierbei parallel zur Gegenstandebene, bzw. Ablagefläche ausgerichtet. Durch den Klassifikator 26, ein neuronales Netzwerk, wird das abgelegte medizinische Instrument 38 einer der beiden Klassen "Ringinstrument" und "kein Ringinstrument" zugeordnet. Das hierfür genutzte neuronale Netzwerk wird speziell auf die beiden Klassen trainiert und ist dann in der Lage, medizinische Instrumente 38 mit und ohne Ringgriffe den beiden zuvor definierten Klassen zuzuordnen.

In einer Entscheidung 52 wird entschieden, ob das medizinische Instrument 38 erkannt wurde. Ist dies der Fall, fährt das Verfahren in einem Schritt 56 fort, in dem eine objektspezifische Weiterverarbeitung des erkannten medizinischen Instrumentes 38 initiiert wird.

Medizinische Instrumente 38 ohne Ringgriffe müssen meist nicht zur Reinigung in einem speziellen Öffnungswinkel geöffnet werden. Ein Ablegen auf einem Reinigungssiebkorb oder weiteren Fördermechanismus reicht hier für einen Weitertransport bzw. die eigentliche Reinigung der medizinischen Instrumente 38 aus. Da das Verfahren zur Wiederaufnahme von medizinischen Instrumenten 38 ohne einem Ringgriff entsprechend einfacher aufgebaut ist, wird dieser Zweig des Verfahrens zuerst beschrieben, der in einem Schritt 58 beginnt.

Aufbauend auf der Berechnung der Greifkoordinate von medizinischen Instrumenten 38 ohne Ringgriff wird im Anschluss der Vorschlag zur Berechnung der Greifkoordinate von medizinischen Instrumenten 38 mit Ringgriff beschrieben und wie diese gezielt ausgerichtet werden, um sie über den oben beschriebenen Mechanismus öffnen zu können.

In einem Schritt 62 wird dazu das aufgenommene Bild aus der Vogelperspektive mit einem Canny-Edge-Algorithmus bearbeitet. Das Resultat davon ist eine Ein-Pixel starke Kantendarstellung der Umrisse des medizinischen Instruments 38. Kantenpixel des medizinischen Instruments 38 werden bevorzugt in schwarz, alle weiteren Pixel in weiß dargestellt. Um die größte zusammenhängende Kontur (die Außenkontur des abgelegten medizinischen Instruments 38) wird ein Rechteck gezogen. In einem nächsten Unterschritt von Schritt 62 wird der Mittelpunkt des Rechtecks in Pixel-Koordinaten errechnet. Um mit dem Greifer 21 in einem späteren Schritt das medizinische Instrument 38 gezielt wiederaufnehmen zu können, wird in horizontaler und in vertikaler Richtung jeweils das erste verfügbare Kantenpixel gesucht. Sobald dieses gefunden wurde, wird dieses als neue Greifkoordinate zur Wiederaufnahme des abgelegten medizinischen Instruments 38 in Pixelkoordinaten definiert.

Der Greifer 21 ist vorteilhafterweise an dem 6-DOF Knickarmroboter 24 montiert, daher muss die errechnete Pixelkoordinate zunächst in Roboterkoordinaten (Tool-Center-point (TCP) des Roboters) transformiert werden. Die Transformation erfolgt über einen zuvor gespeicherten Eckpunkt des Bildausschnitts der Ablagefläche (beispielsweise obere linke Ecke) in Roboterkoordinaten und der Vektoraddition von der Ecke ausgehend zu der neuen Greifkoordinate. Die Vektoren sind dabei in einer metrischen Einheit (Millimeter) bekannt, indem eine Umrechnung der Distanz in Pixel über einen zuvor errechneten konstanten Umrechnungsfaktor erfolgt.

Die Soll- und Greifkoordinaten sind im Terminal 66 bekannt, sodass das Verfahren zu einem Knotenpunkt 70 weitergeht. Zu Beginn des Verfahrens wird in einem Schritt 74 wird eine Datenverbindung zu einer Recheneinheit vorbereitet, die in einem Terminal 74 initiiert wird. Das Verfahren beginnt dann die oben beschriebene Schleife zur Objekterkennung in Schritt 40.

Ausgehend von Schritt 74 wird in einer Entscheidung 78 geprüft, ob Soll-Koordinaten vorhanden sind. Ist dies der Fall, fährt das Verfahren in einem Schritt 82 fort, in welchem die Soll-Koordinaten verarbeitet werden. Hierbei wird die jeweilige errechnete Pixelkoordinate zunächst in Roboterkoordinaten (Tool-Center-point (TCP) des Roboters) transformiert werden.

Der Greifer 21 nimmt das medizinische Instrument 38 in einem Schritt 88 in den errechneten Koordinaten (Mittelpunkt der äußeren Kontur) auf und legt das medizinische Instrument 38 an einer klassenspezifischen Position ab. In einem Terminal 96 wird der Magnet des Greifers 38 angeschaltet. In einem Schritt 102 erfolgt eine objektspezifische Ablage, beispielsweise das Auffädelns eines Stabs an der Sollkoordinate). In einem Terminal 106 wird der Magnet des Greifers 38 ausgeschaltet. Die Variablen x und a sind die Übergabewerte der Koordinaten, die auf die Ebene des TCP (Tool Center Point) bezogen sind.

Falls in der Entscheidung 78 keine Sollkoordinaten vorhanden sind, schreitet das Verfahren in den Terminalen 110, 118 weiter zu einem Schritt 130, in dem der Magnet auf eine Position a des Rasters fährt. In einem Terminal 134 wird der Magnet angeschaltet und in einem Schritt 138 wird ein medizinisches Instrument 38 angehoben und zu einer Scan-Position gefahren. In einer Entscheidung 144 wird der Greiferzustand überprüft. Ist der Greifer 21 leer (d.h. das Fahren zur Scan-Position mit einem angehobenen Instrument war erfolglos) fährt das Verfahren in Terminal 122 fort. Andernfalls verzweigt das Verfahren zu einem Schritt 150. Die Scan-Position entspricht hierbei der Abwurfposition für die Objekterkennung und Bildverarbeitung. In einem Terminal 156 wird dazu zum Abwerfen der Magnet ausgeschaltet.

Sollten durch den ersten starren "Griff in die Kiste" mehrere medizinische Instrumente 38 durch den oben beschriebenen elektromagnetischen Greifer 21 aufgegriffen und auf der Ablagefläche abgelegt werden, wird nach der zuvor beschriebenen Aufnahme des erkannten Instruments 38 ein erneuter Scan der Ablagefläche ohne einen erneuten "Griff in die Kiste" durchgeführt. Der oben beschriebene Algorithmus wird erneut durchgeführt, bis kein medizinisches Instrument 38 mehr auf der Ablagefläche detektiert wird. Zur Erkennung, ob noch ein medizinisches Instrument 38 auf der Ablagefläche liegt, wird ebenfalls der Canny-Edge-Algorithmus eingesetzt. Sofern keine Kanten erkannt werden, wird die Ablagefläche als leer erkannt.

Sollte durch das neuronale Netzwerk ein medizinisches Instrument 38 der Klasse "Ringgriff' erkannt werden (Schritt 56), wird eine erweiterte Berechnung durchgeführt, die in einem Schritt 170 beginnt. Diese wird im folgenden Abschnitt beschrieben. Analog der Berechnung der Klasse "kein Ringgriff' wird auch bei Instrumenten der Klasse Ringgriff in einem Schritt 176 zunächst eine Kantenerkennung mit Hilfe des Canny-Edge Algorithmus durchgeführt und die größte Kontur errechnet.

Die FIG. 2 zeigt eine unbearbeitete Darstellung der optisch erfassten Ablagefläche auf der x-Achse 36 und der y-Achse 37 sind jeweils Pixel-Koordinaten abgetragen. Auf der Ablagefläche liegt ein medizinisches Instrument 38, welches vorliegend eine Schere 39 ist und damit ein Ringgriffinstrument.

Im Gegensatz zu den medizinischen Instrumenten 38 ohne Ringgriff sollen medizinische Instrumente 38 mit Ringgriffen jedoch durch das im Zusammenhang mit FIG. 2 beschriebene Öffnungssystem 1 geöffnet werden. Grundsätzlich sind hierfür folgende weitere Information zusätzlich zur Greifkoordinate für die erneute Aufnahme notwendig:
- Mittelpunkte beider Kreise (Ringgriffe) in Roboterkoordinaten
- Abstand beider Mittelpunkte zueinander, um die beiden Achsen des oben beschriebenen Mechanismus in den gewünschten Abstand zu verfahren
- Gesamtorientierung des Instruments in der Ebene (Gelenk des medizinischen Instruments 38 oberhalb / unterhalb der Ringgriffe), um eine Arretierung bei bspw. Arterienklemmen öffnen zu können (mit Hilfe der Schwenk-Klemmzylinder 7a, 7b)
- Neue Soll-Position der Ringgriffe für einen definierten Öffnungswinkel an der spezifischen Position für medizinische Instrumente 38 der Klasse "Ringgriff'

Die beiden Ringgriffe werden beispielsweise durch ein Open Source Tool wie "O-penCV" mit der bereitgestellten Funktion HoughCircles() detektiert. Hierbei handelt es sich um einen frei zugänglichen Code zur Erkennung von Kreisen in Bildern. Über einen Parameterset kann ein Bereich der zu erkennenden Radien vorgegeben werden. Da Ringgriffe medizinischer Instrumente 38 meist einen ähnlichen Radius besitzen, wird iterativ die notwendige Bandbreite möglicher Radien über das Parameterset justiert. Der Befehl HoughCircles() setzt ein Bild in Graustufen voraus. Da das bereits mit Hilfe des Canny-Edge-Algorithmus kantensegmentierte Bild genutzt wird, ist diese Vorgabe erfüllt.

Grundsätzlich sind Ringgriffe nicht rund, sondern in Ellipsenform. Allerdings ist die Berechnung und Erkennung von Ellipsen weitaus komplexer als die von Kreisen. Daher werden die Teilkreise der oberen und unteren Hälfte beider Ellipsen als Radius erkannt und der Mittelpunkt der Ellipsen entsprechend angenähert. Diese Vereinfachung kann getroffen werden, da die jeweilige mechanische Achse nicht exakt im Mittelpunkt der Ellipsen positioniert werden muss, sondern ein Positionieren innerhalb der Ellipse zum Öffnen der medizinischen Instrumente 38 ausreicht.

Nun ist eine Annäherung der beiden Mittelpunkte beider elliptischen Ringgriffe in der Bild-Ebene bekannt. Basierend auf der Kenntnis dieser beiden Punkte wird die Orientierung des medizinischen Instruments 38 auf der Ablagefläche und die neuen Greifkoordinaten zur Wiederaufnahme mit dem elektromagnetischen Greifer 21 berechnet. Dazu wird zunächst eine Verbindungsgerade zwischen den beiden Mittelpunkten erstellt. Über den Winkel dieser Verbindungsgeraden gegenüber der Horizontalen kann bereits eine erste Information über die Orientierung des medizinischen Instrumentes 38 bereitgestellt werden. Im nächsten Schritt wird auf halber Distanz der Verbindungsgeraden im rechten Winkel zur Geraden in die obere und untere Richtung nach dem ersten verfügbaren Kantenpixel (schwarzer Pixel) gesucht. Bei einem minimal geöffneten Ringgriff-Instrument 38 liegt der erste gefundene Pixel in der Nähe des Gelenks. Dieser errechnete Punkt eignet sich gut, um das medizinische Instrument 38 mit einem elektromagnetischen Greifer 21 erneut stabil aufzunehmen.

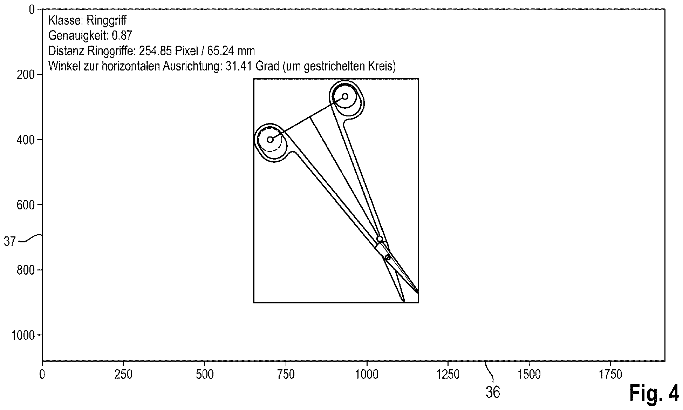

Die Berechnung ist graphisch in FIG. 4 dargestellt, in der auch die erkannten Ecken und die umrahmende Kontur gut erkennbar sind.

In einer Entscheidung 180 wird geprüft, ob durch das oben beschriebene Vorgehen die Ringe erkannt wurden. Ist dies der Fall, sind insgesamt nun die beiden Punkte innerhalb der beiden elliptischen Ringgriffe, der Orientierungswinkel des medizinischen Instruments 38 auf der Fläche sowie eine neue Greifkoordinate in Pixelkoordinaten bekannt und das Verfahren schreitet in Terminal 66 fort. Über die Information, ob die neue Greifkoordinate ober- oder unterhalb der Geraden liegt, kann außerdem entnommen werden, wo sich die Spitze des medizinischen Instruments 38 befindet. Es sind somit alle Informationen bekannt, um das Instrument mit dem elektromagnetischen Greifer 21 aufzunehmen und die Achsen 3, 5 zum Öffnen über die Ringgriffe in den errechneten Abstand (Länge der Verbindungsgeraden) zu verfahren und innerhalb der Ellipsen zu positionieren. Die Umrechnung der Pixelkoordinaten in Roboterkoordinaten erfolgt analog zu der Vorgehensweise der Klasse "kein Ringgriff".

Sofern in der Entscheidung 180 keine Ringe erkannt wurden, verzweigt das Verfahren zu einem Schritt 186, in welchem ein Scan nach Restkonturen erfolgt. Wenn keine Restkonturen mehr erkannt werden, wird ein neuer Siebkorb bzw. Instrumente geliefert und der Algorithmus startet erneut. In einer Entscheidung 192 wird geprüft, ob Restkonturen erkannt wurden. Ist dies der Fall, verzweigt das Verfahren zu einem Schritt 196, in welchem mit Hilfe des Canny-Edge-Algorithmus eine Kantenerkennung durchgeführt wird und der Mittelpunkt der größtem umrahmten Kontur bestimmt wird, welcher die Soll-Koordinaten für den Greifer 21 darstellt. Das Verfahren fährt dann mit Terminal 66 fort. Sofern in der Entscheidung 192 keine Restkonturen erkannt wurden, wird in einem Schritt 200 die Schleife der Objekterkennung beendet, in einem Terminal 204 liegen keine Soll-Koordinaten vor. Die Master-Slave-Architektur aus FIG. 5 repräsentiert die Kommunikation der beiden Roboter und der Sensorik miteinander.

### Bezugszeichenliste

- 1: Öffnungssystem
- 2: Basis
- 3: Achse
- 4: Spindel
- 5: verschiebbare Achse
- 6: Schlitten
- 7a, 7b: Schwenk-Klemmzylinder
- 8a, 8b: Klemmelement
- 9a, 9b: Grundelement der Achsenaufnahme
- 10: Getriebe
- 11: Elektroantrieb
- 12: Aktor
- 13a, 13b: Schwenk-Klemmvorrichtung
- 14: Schiene
- 20: Greifsystem
- 21: Greifer
- 22: Objekterkennungsvorrichtung
- 23: 6-DOF-Einheit
- 24: 6-DOF-Knickarmroboter
- 25: Steuer- und Regeleinheit
- 26: Klassifikator
- 36: x-Achse
- 37: y-Achse
- 38: medizinisches Instrument
- 39: Schere
- 40: Schritt
- 46: Terminal
- 52: Entscheidung
- 56: Schritt
- 58: Schritt
- 62: Schritt
- 66: Terminal
- 70: Knotenpunkt
- 74: Schritt
- 78: Entscheidung
- 82: Schritt
- 88: Schritt
- 96: Terminal
- 102: Schritt
- 106: Terminal
- 110: Terminal
- 114: Terminal
- 118: Terminal
- 122: Terminal
- 130: Schritt
- 134: Terminal
- 138: Schritt
- 144: Entscheidung
- 150: Schritt
- 156: Terminal
- 170: Schritt
- 176: Schritt
- 180: Entscheidung
- 186: Schritt
- 192: Entscheidung
- 196: Schritt
- 200: Schritt
- 204: Terminal

## Patentansprüche

1. Öffnungssystem (1) zum Öffnen und/oder Entriegeln eines medizinischen, insbesondere chirurgischen, Instrumentes (38) mit Ringgriffen, umfassend
- eine Basis (2);
- zwei auf der Basis (2) angeordnete und mittels eines Aktors (12) gegeneinander verschiebbare Achsen (3, 5), die jeweils auf flächigen Grundelementen (9a, 9b) angeordnet sind, welche zur Basis (2) einen unterschiedlichen Abstand aufweisen;
- zwei Schwenk-Klemmvorrichtungen (13a, 13b), welche jeweils ein Klemmelement (8a, 8b) zum Klemmen eines auf eine Achse (3,5) eingefädelten Ringgriffes mit dem Grundelement (9a, 9b) umfassen.

2. Öffnungssystem (1) nach Anspruch 1, wobei die jeweilige Schwenk-Klemmvorrichtung (13a, 13b) als Schwenk-Klemmzylinder (7a, 7b) ausgebildet ist.

3. Öffnungssystem (1) nach Anspruch 1 oder 2, wobei die wenigstens eine Schwenk-Klemmvorrichtung (13a, 13b) ein mit Hilfe eines Antriebs verfahrbares Grundelement (9a, 9b) umfasst.

4. Öffnungssystem (1) nach einem der vorherigen Ansprüche, wobei auf der Basis (2) eine Schiene (14) angeordnet ist, auf welcher ein Schlitten (6) verfahrbar ist, auf welchem eine der Achsen (5) und eine Schwenk-Klemmvorrichtung (13b) angeordnet sind.

5. Öffnungssystem (1) nach Anspruch 4, wobei der Schlitten (6) mittels einer durch den Aktor (12) drehbaren Spindel (4) entlang der Schiene (14) verfahrbar ist.

6. Öffnungssystem (1) nach einem der vorherigen Ansprüche, wobei der Aktor (12) einen Elektroantrieb (11) und ein Getriebe (10) umfasst.

7. Greifsystem (20) zum Aufnehmen und Handhaben von medizinischen, insbesondere chirurgischen, Instrumenten (38), umfassend
- einen Greifer (21) zum Anheben und Ablegen des medizinischen Instruments (38);
- eine Objekterkennungsvorrichtung (22);
- ein Öffnungssystem (1) nach einem der vorherigen Ansprüche,
- eine Steuer- und Regeleinheit (25),
wobei die Objekterkennungsvorrichtung (22) zum Erkennen eines medizinischen Instrumentes (38) mit Ringgriffen ausgebildet ist, und wobei das Greifsystem (20) ausgebildet ist, das medizinische Instrument (38) mit Ringgriffen in das Öffnungssystem (1) einzulegen.

8. Greifsystem (20) nach Anspruch 7, wobei die Objekterkennungsvorrichtung (22) einen Klassifikator (26) umfasst, welcher insbesondere als neuronales Netz ausgebildet ist, zum Klassifizieren des medizinischen Instrumentes (38) in die Klassen "weist Ringriffe auf" und "weist keine Ringgriffe auf".

9. Greifsystem (20) nach Anspruch 8, wobei der Klassifikator (26) ausgebildet ist, einen Verriegelungsmechanismus des medizinischen Instruments (38) zu erkennen, und wobei die Steuer- und Regeleinheit (25) den Greifer (21) aufgrund des erkannten Verriegelungsmechanismus derart ansteuert, dass das medizinische Instrument (38) in einer Entriegelungsorientierung in das Öffnungssystem (1) eingelegt wird.

10. Greifsystem (20) nach einem der Ansprüche 7 bis 9, umfassend eine 6-DOF-Einheit (23), an welcher die Basis (2) des Öffnungssystem (1) montiert ist.

11. Greifsystem (20) nach einem der Ansprüche 7 bis 10, umfassend einen 6-DOF-Knickarmroboter (24), an welchem der Greifer (21) montiert ist.

12. Verfahren zum Betreiben eines Greifsystems (20) nach einem der Ansprüche 7 bis 11, umfassend die Schritte
- Greifen eines medizinischen Instrumentes (38) und Ablegen des Instrumentes (38) auf einer Ablagefläche und/oder Positionieren des Instrumentes (38) vor einer Referenzfläche;
- Optisches Erfassen der Konturen des medizinischen Instrumentes (38) und Erkennen, ob das medizinische Instrument (38) Ringgriffe aufweist, und sofern dies der Fall ist, Positionieren des medizinischen Instrumentes (38) in dem Öffnungssystem (1) und Öffnungen und/oder Entriegeln des medizinischen Instrumentes (38) durch das Öffnungssystem (1);
- Erneutes Greifen des medizinischen Instrumentes (38) und Ablegen des medizinischen Instrumentes (38) an einer vorgegeben Stelle.

13. Verfahren nach Anspruch 12, wobei die Konturen des medizinischen Instrumentes (38) mit Hilfe eines Kantenerkennungsalgorithmus erkannt werden.

14. Verfahren nach Anspruch 12 oder 13, wobei die Objekterkennungsvorrichtung (22) Merkmale auf dem medizinischen Instrument (38) erkennt und darauf basierend einen Output generiert.

15. Verfahren nach einem der Ansprüche 12 bis 14, wobei das Greifen entlang eines vorgegebenen Rasters erfolgt.
